# EUROPÄISCHE PATENTSCHRIFT

(11) **EP 3 277 239 B1**
(45) Veröffentlichungstag und Bekanntmachung des Hinweises auf die Patenterteilung: **30.12.2020**
(21) Anmeldenummer: 16713489.9
(22) Anmeldetag: 04.04.2016
(51) Int. Cl.: A61F 13/00, A61L 15/46

(54) **WUNDAUFLAGE ZUR WUNDBEHANDLUNG IM FEUCHTEN ODER FEUCHTNASSEN MILIEU**
WOUND DRESSING FOR WOUND TREATMENT IN A DAMP OR WET ENVIRONMENT
PANSEMENT POUR LE TRAITEMENT D'UNE PLAIE EN MILIEU HUMIDE OU MOUILLÉ

(30) Priorität: 02.04.2015 DE 102015206083
(43) Veröffentlichungstag der Anmeldung: 07.02.2018
(73) Patentinhaber: IVF Hartmann AG, 8212 Neuhausen am Rheinfall (CH)
(72) Erfinder: MARZ, Jacqueline, 78239 Rielasingen-Worblingen (DE); ROTHMAIER, Markus, 8308 Illnau (CH)
(74) Vertreter: DREISS Patentanwälte PartG mbB
(86) Internationale Anmeldenummer: PCT/EP2016/057364
(87) Internationale Veröffentlichungsnummer: WO 2016/156619

(56) Entgegenhaltungen:
- EP-B1- 0 594 034
- WO-A2-2009/019229
- DE-A1-102010 020 050

## Beschreibung

Die Erfindung betrifft eine Wundauflage zur Wundbehandlung im feuchten oder feuchtnassen Milieu, mit einem faservliesbasierten Saug-/Spülkörper, in dem superabsorbierendes Material verteilt aufgenommen ist, wobei der Saug-/Spülkörper herstellerseitig mit einer salzhaltigen wässrigen Lösung, insbesondere Ringerlösung, die vorzugsweise eine Substanz mit antimikrobieller Wirkung enthält, beaufschlagt ist, und mit einer die äußeren Sichtseiten der Wundauflage bildenden Umhüllung, wobei die Umhüllung auf der wundzugewandten Seite der Wundauflage eine Schicht aus einem textilen Flächenmaterial, insbesondere aus einem Gestrick, Gewirke oder Gewebe, umfasst, wobei die Schicht aus einem textilen Flächenmaterial auf der wundzugewandten Seite eine außenseitig partiell und strukturiert aufgebrachte atraumatisch wirkende Beschichtung, vorzugsweise aus Silikon, mit einem Bedeckungsgrad von höchstens 70 % aufweist.

Eine derartige Wundauflage ist aus WO 2011/141454 A1 der Anmelderin bekannt. Es handelt sich hierbei um eine wundkissenartige oder kompressenartige Wundauflage, die auf eine Wunde aufbringbar ist oder auch zum Austamponieren tiefer Wunden verwendet werden kann. Der Saug-/Spülkörper wird herstellerseitig, insbesondere bis zur Sättigung mit einer salzhaltigen wässrigen Lösung beaufschlagt, welche das superabsorbierende Material quellen und in einen gelartigen Zustand übergehen lässt. Dies verleiht dem Saug-/Spülkörper eine duale Funktion bei Wunden mit starker Exsudation. Wundsekrete werden einschließlich ihrer kritischen Bestandteile wie Keime durch den Saug-/Spülkörper aktiv aufgenommen und darin gehalten, wobei der Saug-/Spülkörper im Gegenzug die salzhaltige wässrige Lösung an die Wunde abgibt und somit ein feuchtes Wundmilieu schafft oder unterstützt. Hierdurch wird die Wundreinigung und eine positive Wundkonditionierung unterstützt und somit die Heilung positiv beeinflusst. Man spricht hier von interaktiver Nasstherapie, die insbesondere bei schlecht heilenden Wunden, bei klinisch manifest infizierten Wunden oder bei chronischen Wunden mit unterschiedlicher Genese, wie diabetisches Gangrän, Dekubitalulcus oder Ulcus cruris bevorzugt Anwendung findet. Aus EP 0 594 034 B1 war ferner bereits eine Wundauflage mit einem Saug-/Spülkörper, der einen Superabsorber enthält oder aus einem Superabsorber besteht, und mit einer zumindest auf der wundzugewandten Seite feuchtigkeitsdurchlässigen Hüllschicht aus einem textilen Material bekannt.

Bei der vorerwähnten Ringerlösung handelt es sich typischerweise um eine wässrige Lösung mit Natriumchlorid, Kaliumchlorid und Calciumchlorid (insbesondere 8,6 g NaCl, 0,3 g KCl und 0,33 g CaCl₂ je Liter).

Die Wechselintervallszeit, d.h. die Verwendungszeit einer Wundauflage bis zum nächsten Verbandwechsel, soll wenigstens 24 h betragen, wobei Bestrebungen bestehen, die Wechselintervallszeit zu erhöhen, insbesondere auf 48 bis 72 h. Dies wäre aus betriebswirtschaftlichen Gesichtspunkten, aber auch aus Gründen einer durch häufige Verbandwechsel verursachten Störung der Wundheilung wünschenswert.

US 2008/0082059 A1 erwähnt eine in einen Unterdruckwundverband integrierte Wundauflage mit einem Saugkörper, für den in verschiedenen Listen eine Vielzahl von Stoffen und Stoffzusätzen genannt sind, insbesondere eine Mehrzahl von Trägerstoffen, eine Mehrzahl von Stoffzusätzen, darunter Polyhexamethylene biguanide und isotonische Kochsalzlösung. Eine konkrete Zusammensetzung des Saugkörpers und seiner Umhüllung ist nicht offenbart. WO 2010/024928A1 offenbart einen Verbund umfassend eine Filmschicht, eine Kollagenschicht und eine Hydrogelschicht mit Polyhexamethylenbiguanid. WO 2003/039602 A2 lehrt bei einem Material zur Aufnahme von Wundexudat antimikrobiell wirkende Gruppen kovalent an ein Trägerpolymer zu binden und hierdurch zu lokalisieren.

Der vorliegenden Erfindung liegt die Aufgabe zugrunde, eine Wundauflage der eingangs beschriebenen Art im Hinblick auf ihre wirtschaftliche Herstellbarkeit zu verbessern. Ferner soll ihre Drapierfähigkeit und Biegsamkeit verbessert werden.

Diese Aufgabe wird bei einer Wundauflage erfindungsgemäß dadurch gelöst, dass die Umhüllung auf der wundabgewandten Seite der Wundauflage eine Faservliesschicht umfasst, welche die wundabgewandte äußere Sichtseite der Wundauflage bildet, und dass auf der wundzugewandten Seite dieser Faservliesschicht eine flüssigkeitsundurchlässige Kunststofffilmschicht angeordnet ist, und dass die Faservliesschicht, die Kunststofffilmschicht, der Saug-/Spülkörper und die Schicht aus textilem Flächenmaterial nicht flächenhaft miteinander verbunden sind, sondern mit ihren Flachseiten lose und verschieblich gegeneinander anliegen, wobei die Faservliesschicht, die Kunststofffilmschicht, der Saug-/Spülkörper und die Schicht aus textilem Flächenmaterial nur entlang ihres Umfangsrands durch eine Fügeverbindung miteinander verbunden sind.

Dadurch, dass die wundabgewandte äußere Sichtseite der Wundauflage nicht wie bei dem vorbekannten Produkt von einer Kunststofffilmschicht, sondern von einer Faservliesschicht gebildet wird, ist die taktile und haptische Ergreifbarkeit der Wundauflage, auch mittels Instrumenten, verbessert, da die Faservliesschicht weniger glitschig ist als eine Kunststofffilmschicht, sondern durchaus auch im nassen Zustand als griffig zu bezeichnen ist. Ferner wird die Anmutung, insbesondere im Kontakt mit den Fingern des Pflegepersonals und mit der Körperoberfläche des Patienten verbessert und als angenehmer empfunden. Weiter erweist es sich als vorteilhaft, dass die Biegsamkeit und die Drapierbarkeit der erfindungsgemäßen Wundauflage verbessert ist. Es zeigte sich überraschenderweise, dass die nicht außen sondern auf der wundzugewandten Seite der Faservliesschicht angeordnete Kunststofffilmschicht die Biegsamkeit und Drapierbarkeit der Wundauflage nicht behindert, was auch darauf zurückgeführt wird, dass die Schichten nicht flächenhaft sondern nur entlang ihres Rands miteinander verbunden sind, so dass sie erfindungsgemäß mit ihren Flachseiten nur lose und verschieblich gegeneinander anliegen. Weiter hat es sich als besonders vorteilhaft erwiesen, dass die bestimmungsgemäß unlösbare Verbindung der Schichten nur entlang ihres Umfangsrands zugleich zum Vereinzeln der einzelnen Wundauflagen aus größeren Flachmaterialanordnungen im Zuge der Herstellung eingesetzt werden kann. Die Herstellung dieser randseitigen Fügeverbindung zwischen vorzugsweise allen Schichten innerhalb der Wundauflage wird also zugleich zur Herstellung des Trenn- oder Vereinzelungsschnitts oder -vorgangs genutzt.

Diese nur randseitig vorgesehene Fügeverbindung zwischen den Schichten kann in vorteilhafter Weise von einer Ultraschallschweißverbindung oder einer Laserschweißverbindung gebildet sein. Die Ultraschall- oder Laserschweißverbindung kann eine Aufeinanderfolge von diskreten Schweißstellen umfassen. Diese Schweißstellen können aber auch derart quasikontinuierlich aufeinanderfolgend angeordnet sein, dass visuell der Eindruck einer kontinuierlich durchgehenden Verbindungslinie entsteht.

Die erwähnte sichtseitige Faservliesschicht kann aus thermoplastischem Fasermaterial, insbesondere aus Polyolefin, insbesondere aus Polypropylen, gebildet sein.

Das Flächengewicht der Faservliesschicht auf der wundabgewandten Seite der Wundauflage beträgt vorzugsweise 15-100 g/m², bevorzugt 20-60 g/m², besonders bevorzugt 25-40 g/m². Es kann vorteilhaft sein, als Faservliesschicht zwei insbesondere identische Materiallagen eines Faservliesmaterials übereinander zu legen, bevorzugt mit einem Flächengewicht von jeweils 25-40 g/m². Auf diese Weise kann die Prozesssicherheit beim Bilden der randseitigen Fügeverbindung gesteigert werden.

Weiter erweist es sich als vorteilhaft, wenn die Dichte der Faservliesschicht auf der wundabgewandten Seite der Wundauflage 10-1000 kg/m³, bevorzugt 50-250 kg/m³, besonders bevorzugt 100-150 kg/m³ beträgt.

Weiter erweist es sich als vorteilhaft, wenn die flüssigkeitsundurchlässige Kunststofffilmschicht aus einem thermoplastischen Material, insbesondere aus Polyolefin, insbesondere aus Polypropylen, gebildet ist.

Weiter erweist es sich als vorteilhaft, wenn die flüssigkeitsundurchlässige Kunststofffilmschicht ein Flächengewicht von 5-100g/m², bevorzugt 8-50 g/m², besonders bevorzugt 10-25 g/m² und eine Dicke von 5-100 µm, bevorzugt 8-50 µm, besonders bevorzugt 10-25 µm aufweist.

Es erweist sich als vorteilhaft, wenn der faservliesbasierte Saug-/Spülkörper cellulosische Fasern, insbesondere eine Mischung aus cellulosischen Fasern und thermoplastischen Fasern, insbesondere Polyolefin-Fasern, insbesondere Polypropylen-Fasern oder Polypropylen/Polyethylen-Fasern, umfasst.

Dabei beträgt das Flächengewicht des Faseranteils des Saug-/Spülkörpers vorteilhafterweise 20 bis 500 g/m², bevorzugt 30-300 g/m², besonders bevorzugt 30-200 g/m², insbesondere 30 - 150 g/m².

Es erweist sich als vorteilhaft, wenn die Dichte des Faseranteils des Saug/Spülkörpers 20-500 kg/m³, bevorzugt 30-300 kg/m³, besonders bevorzugt 50-200 kg/m³ beträgt. Weiter erweist es sich als vorteilhaft, wenn die Schicht aus textilem Flächenmaterial aus einem thermoplastischen Material, insbesondere aus Polyolefin, insbesondere aus Polypropylen, gebildet ist.

Weiter erweist es sich als vorteilhaft, wenn der Bedeckungsgrad der partiell und strukturiert aufgebrachten atraumatisch wirkenden Beschichtung 20 - 70 %, insbesondere 25 - 50 %, insbesondere 30 - 40 % beträgt.

Es erweist sich als weiter vorteilhaft, wenn die partiell und strukturiert aufgebrachte atraumatisch wirkende Beschichtung streifenförmig ausgebildet ist. Die Streifen können dabei linear erstreckt sein. Sie verlaufen vorzugsweise parallel oder äquidistant zueinander. Die Breite eines Streifens beträgt vorteilhafterweise 1 bis 3 mm. Der Abstand der Streifen voneinander beträgt vorteilhafterweise 4 bis 8 mm, insbesondere 4 bis 6 mm.

Weiter erweist es sich als vorteilhaft, wenn die Wundauflage derart ausgebildet ist, dass das superabsorbierende Material anionisch ist und negative Gruppen aufweist und dass die wässrige Lösung eine Substanz mit antimikrobieller Wirkung umfasst, bei der es sich um Silberkationen, Biguanid oder Biguanid-Derivate, Polyguanidine, *N*-Octyl-1-[10-(4-Octyliminopyridin- 1-yl)decyl]pyridin-4-imin (Octenidin), quartäre Ammoniumverbindungen, Triazine oder die Ammoniumverbindung Taurolidin handelt, und dass diese Substanz mit antimikrobieller Wirkung bei pH-Werten von 4 - 7,5 eines typischen feuchten oder feuchtnassen Wundmilieus kationisch geladen vorliegt und daher von negativen Gruppen des anionischen superabsorbierenden Materials attraktiv angezogen wird und so innerhalb des Saug-/Spülkörpers antimikrobiell wirkt. Das Flächengewicht des superabsorbierende Materials innerhalb des Saug/Spülkörpers beträgt vorzugsweise 30 - 150 g/m², insbesondere 50 - 100 g/m², insbesondere 60 - 80 g/m².

Nach einem weiteren Erfindungsgedanken von besonderer Bedeutung wird vorgeschlagen, die Wundauflage so auszubilden, dass die Dicke der Wundauflage im mit salzhaltiger wässrigen Lösung beaufschlagten Zustand 3 - 7 mm, insbesondere 4 - 6 mm beträgt.Es wird hierbei davon ausgegangen, dass die Dicke der wundabgewandten Faservliesschicht und der darunter befindlichen Kunststofffilmschicht sowie die Dicke der wundzugewandten Schicht aus textilem Flächenmaterial mit der partiell und strukturiert aufgebrachten atraumatischen Beschichtung höchstens 1 mm der Gesamtdicke der herstellerseitig befeuchteten Wundauflage ausmachen, so dass der Rest von dem flüssigkeitsspeichernden Saug-/Spülkörper herrührt. Gegenüber vorbekannten Wundauflagen für die feuchtnasse Wundtherapie ist die wie vorstehend ausgebildete Wundauflage mit deutlich geringerer Dicke ausgebildet. Es wurde überraschenderweise festgestellt, dass das Absorptionsverhalten für Wundexsudate aber nicht herabgesetzt ist. Eine mögliche, aber noch nicht belegte Erklärung hierfür wäre, dass für die Absorption von Wundexsudat in dem mit salzhaltiger wässriger Lösung aktivierten Saug-/Spülkörper im Wesentlichen eine Oberflächenschicht des Saug-/Spülkörpers verantwortlich ist. Diese an sich überraschende Eigenschaft eröffnet die Möglichkeit, mit geringeren Flächengewichten der Komponenten des Saug-/Spülkörpers zu arbeiten, was wiederum die Dicke der Wundauflage insgesamt reduziert und ihre Biegsamkeit und Drapierfähigkeit verbessert.

Es wird weiter Schutz beansprucht für ein Verfahren zum Herstellen einer erfindungsgemäßen Wundauflage, welches dadurch gekennzeichnet ist, dass die Faservliesschicht, die flüssigkeitsundurchlässige Kunststofffilmschicht, eine den faservliesbasierten Saug-/Spülkörper bildende Schicht und die Schicht aus textilem Flächenmaterial als jeweils endlose Flachmaterialien zugeführt und übereinander in der genannten Schichtenfolge angeordnet werden und dass die Schichten nur entlang ihres Umfangsrands miteinander verbunden werden und dabei die Wundauflagen aus den Flachmaterialien vereinzelt werden.

Wie eingangs bereits erwähnt, kann das Verbinden der Schichten in vorteilhafter Weise durch Ultraschall- oder Laserschweißen durchgeführt werden.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:
Figur 1 eine schematische Schnittansicht einer erfindungsgemäßen Wundauflage;
Figur 2 eine Ansicht der Wundauflage mit Blick auf die wundzugewandte Seite der Wundauflage.

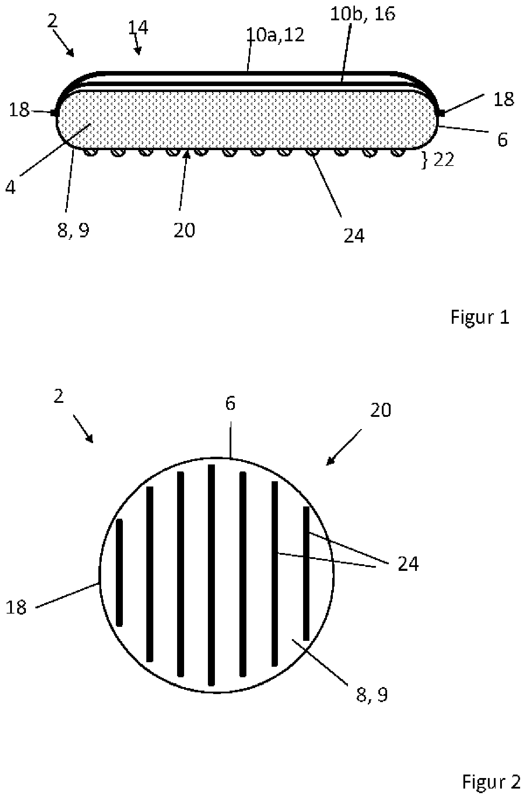

Figur 1 zeigt im Schnitt eine Wundauflage 2. Sie umfasst einen Saug-/Spülkörper 4 auf Faservliesbasis. Bei dieser Faserbasis handelt es sich um eine bevorzugte Mischung aus luftgelegten Zellulosefasern (Zellstoff) und Polypropylen-Fasern oder Polypropylen/Polyethylen-Fasern. Dieser Fasermischung sind superabsorbierende Polymermaterialien (SAP) in Partikelform oder in Faserform möglichst homogen beigemischt, wobei der SAP-Anteil an der Gesamtmasse des Saug-/Spülkörpers 4 vorzugsweise 40 - 50 Gew.-% beträgt. Die mittlere Korngröße der SAP-Partikel liegt beispielsweise bei 150 bis 850 µm (beispielsweise Polyacrylat der Marke Favor pac 300 von der Firma Evonik Stockhausen GmbH).

Der Saug-/Spülkörper 4 ist von einer die Außenseiten der Wundauflage bildenden Umhüllung 6 umgeben, die von einer wundzugewandten Hüllschicht 8 und zwei wundabgewandten Hüllschichten 10a, b gebildet ist. Bei der wundzugewandten Hüllschicht 8 handelt es sich vorzugsweise um eine Schicht 9 aus einem textilen Flachmaterial, wie ein Gestrick, vorzugsweise aus Polypropylen, wobei auch ein Gewebe oder Gewirke in vorteilhafter Weise denkbar wäre, also eine Hüllschicht aus Fäden oder Filamenten mit textiler Bindung, die einen guten Flüssigkeitsaustausch zwischen dem Saug-/Spülkörper 4 und der Wundumgebung gestattet.

Die eine wundabgewandte Hüllschicht 10a ist eine Faservliesschicht 12, vorzugsweise aus Polypropylen, welche eine wundabgewandte Sichtseite 14 der Wundauflage 2 bildet. Die zweite Hüllschicht 10b ist von einer flüssigkeitsundurchlässigen Kunststofffilmschicht 16 gebildet, die unmittelbar unterhalb der Faservliesschicht 12, also auf der wundzugewandten Seite der Faservliesschicht 12, zwischen der Faservliesschicht 12 und dem Saug-/Spülkörper 4 angeordnet ist. Diese beiden wundabgewandten Hüllschichten 10a, 10b sind nicht flächenhaft miteinander verbunden, sie bilden also kein Laminat im eigentlichen Sinne, sondern sie liegen lose und verschieblich flächenhaft aneinander an, wobei sie aber entlang eines Umfangsrands 18 oder eines Umfangsrandbereichs miteinander und mit den weiteren Komponenten der Wundauflage verbunden sind. Hierfür werden die Schichten der Wundauflage als jeweils in einer Maschinenrichtung endlose Flachmaterialien zugeführt und übereinander angeordnet. Es sind dies also die Faservliesschicht 12, die Kunststofffilmschicht 16, eine Faservliesschicht mit superabsorbierenden Polymermaterialien, welche den jeweiligen faservliesbasierte Saug-/Spülkörper bildet, und die wundzugewandte Schicht aus einem textilen Flächenmaterial. Zum Abgrenzen, Definieren und schließlich Vereinzeln der Wundauflage-Produkte wird senkrecht zur Schichtenebene eine alle Schichten erfassende Fügeverbindung, vorzugsweise in Form einer Ultraschallschweißverbindung, hergestellt. Im Zuge der Herstellung dieser Ultraschallschweißverbindung, oder daran anschließend, jedoch entlang des Fügebereichs dieser Verbindung, werden die einzelnen Wundauflage-Produkte dann vereinzelt. Die Breite der Fügeverbindung quer zu ihrer Längserstreckung ist vorzugsweise sehr gering, so dass am fertigen vereinzelten Produkt ein kaum wahrnehmbarer seitlich geringfügigst vorstehender Produktrand wahrnehmbar ist. Wenn die Wundauflage schließlich mit salzhaltiger wässriger Lösung, insbesondere Ringerlösung, herstellerseitig beaufschlagt wird, so wird durch Quellen des Saug-/Spülkörpers 4 die wundabgewandte Hüllschicht 8 und die wundzugewandten Hüllschichten 10a, b voneinander weg gedehnt, so dass der Eindruck eines sehr flachen randständig verrundeten Wundkissens entsteht.

Vor der Beaufschlagung mit salzhaltiger wässriger Lösung wird auf der wundzugewandten Außenseite 20 der wundzugewandten Hüllschicht 8 eine partiell und strukturiert aufgebrachte atraumatisch wirkende Beschichtung 22 vorgesehen. Diese Beschichtung 22 ist vorzugsweise eine Silikonbeschichtung, wobei die Beschichtung porös und im beispielhaft dargestellten Fall von einer Mehrzahl von verhältnismäßig dünnen Streifen 24 oder Linien oder inselförmigen Bereichen gebildet ist, die durch nicht beschichtete Bereiche voneinander separiert sind. In diesen nicht beschichteten Bereichen ist die wundzugewandte Hüllschicht 8 zur Wunde exponiert. Die atraumatisch wirkende Beschichtung 22 bildet diesbezüglich einen Überstand in der in der Beschreibungseinleitung genannten Größenordnung, wodurch einerseits ein Verkleben der Hüllschicht 8 mit Wundgewebe verhindert werden kann und andererseits ein gewisser geringer Abstand zwischen der wundzugewandten Hüllschicht 8 und dem Wundgewebe beibehalten werden kann, wodurch das poröse Hüllschichtmaterial dreidimensional offen bleibt und einen geringeren Widerstand für den Flüssigkeitsdurchtritt in beiden Richtungen über die Gebrauchsdauer der Wundauflage bereitstellt oder behält.

Die vorstehend beschriebene atraumatisch wirkende Beschichtung kann entweder vor oder nach dem Vereinzeln der Wundauflage-Produkte bei der wundzugewandten Hüllschicht 8 vorgesehen werden. Hierfür kann eine Dispensieranlage der in WO 2011/141454 A1 beschriebenen Art verwendet werden. Erst danach werden die dann vereinzelten Wundauflagen mit salzhaltiger wässriger Lösung beaufschlagt. Dieser salzhaltigen Lösung ist eine Substanz mit antimikrobieller Wirkung zugegeben, welche im feuchten oder feuchtnassen Wundmilieu bei pH-Werten im leicht sauren bis neutralen Bereich von pH 4 bis 7,5 kationisch vorliegt. Diese kationische Substanz mit antimikrobieller Wirkung wird von negativen Gruppen des anionischen superabsorbierenden Materials derart angezogen, dass sie auch im flüssigkeitsaustauschenden Betrieb des Saug-/Spülkörpers 4 an die superabsorbierenden Materialien gebunden bleibt, also weitestgehend nicht in das Wundmilieu abgegeben wird. Hierdurch werden mit Wundsekret in den Saug-/Spülkörper 4 eingebrachte Keime an einer Vermehrung gehindert, wodurch eine Rückkontamination in Richtung auf die Wunde weitestgehend verhindert wird. Es wurde festgestellt, dass so eine mikrobiologische Rückkontamination über 72 h weitestgehend verhindert werden konnte, was darauf zurückgeführt wird, dass die antimikrobiell wirkende Substanz aufgrund ihres kationischen Zustands gleichmäßig verteilt innerhalb des Saug-/Spülkörpers 4 in Anbindung an die superabsorbierenden Materialien gehalten wird.

Bei einer beispielhaften bevorzugten Zusammensetzung der Wundauflage 2 besteht die Faservliesbasis des Saug-/Spülkörpers 4 aus 33 g/m² Zellulosefasern (Zellstoff), 11 g/m² Polypropylen/Polyethylen-Fasern als Bindefasern. Dieser Fasermischung sind 70 g/m² der oben genannten superabsorbierenden Polymermaterialien (SAP) homogen beigemischt. Die so erhaltene Mischung (Faservliesbasis + SAP), welche den Saug/Spülkörper 4 bildet, kann zudem von einer zellulosischen Tissueschicht, insbesondere eines Flächengewichts von beispielhaft 17 g/m² auf jeder Seite, umgeben sein (in der Figur nicht dargestellt); dies ist jedoch nicht zwingend erforderlich. Die Wundauflage kann beispielhaft rund ausgebildet sein, mit einer beispielhaften Abmessung des Saug/Spülkörpers 4, die im wesentlichen der Abmessung der Wundauflage 2 entspricht, von 5,5 cm Durchmesser. Die Wundauflage 2 wurde schließlich mit 8 ml Ringerlösung aktiviert, was vorliegend im wesentlichen einer Sättigung des Saug-/Spülkörpers mit Flüssigkeit entspricht. Die Umhüllung 6 ist wie vorausgehend beschrieben ausgebildet.

Figur 2 verdeutlicht den partiellen und strukturierten Auftrag der atraumatischen Beschichtung 22, der hier streifenförmig, also in Form von Streifen 24, mit den eingangs angegebenen Parametern aufgebracht ist.

## Patentansprüche

1. Wundauflage (2) zur Wundbehandlung im feuchten oder feuchtnassen Milieu, mit einem faservliesbasierten Saug-/Spülkörper (4), in dem superabsorbierendes Material verteilt aufgenommen ist, wobei der Saug-/Spülkörper (4) herstellerseitig mit einer salzhaltigen wässrigen Lösung, insbesondere Ringerlösung, die vorzugsweise eine Substanz mit antimikrobieller Wirkung enthält, beaufschlagt ist, und mit einer die äußeren Sichtseiten der Wundauflage bildenden Umhüllung (6), wobei die Umhüllung (6) auf der wundzugewandten Seite der Wundauflage eine Schicht (9) aus einem textilen Flächenmaterial, insbesondere aus einem Gestrick, Gewirke oder Gewebe, umfasst, wobei die Schicht (9) aus einem textilen Flächenmaterial auf der wundzugewandten Seite eine außenseitig partiell und strukturiert aufgebrachte atraumatisch wirkende Beschichtung (22), vorzugsweise aus Silikon, mit einem Bedeckungsgrad von höchstens 70 % aufweist, **dadurch gekennzeichnet, dass** die Umhüllung (6) auf der wundabgewandten Seite der Wundauflage eine Faservliesschicht (12) umfasst, welche die wundabgewandte äußere Sichtseite der Wundauflage bildet, und dass auf der wundzugewandten Seite dieser Faservliesschicht (12) eine flüssigkeitsundurchlässige Kunststofffilmschicht (16) angeordnet ist, und dass die Faservliesschicht (12), die Kunststofffilmschicht (16), der Saug-/Spülkörper (4) und die Schicht (9) aus textilem Flächenmaterial nicht flächenhaft miteinander verbunden sind, sondern mit ihren Flachseiten lose und verschieblich gegeneinander anliegen, wobei die Faservliesschicht (12), die Kunststofffilmschicht (16), der Saug-/Spülkörper (4) und die Schicht (9) aus textilem Flächenmaterial nur entlang ihres Umfangsrands (18) durch eine Fügeverbindung miteinander verbunden sind.

2. Wundauflage nach Anspruch 1, **dadurch gekennzeichnet, dass** die Fügeverbindung eine Ultraschallschweißverbindung ist.

3. Wundauflage nach Anspruch 2, **dadurch gekennzeichnet, dass** die Ultraschallschweißverbindung eine Aufeinanderfolge von diskreten Ultraschallschweißstellen umfasst.

4. Wundauflage nach einem oder mehreren der vorstehenden Ansprüche, **dadurch gekennzeichnet, dass** die Faservliesschicht (12) aus thermoplastischem Fasermaterial, insbesondere aus Polyolefin, insbesondere aus Polypropylen, gebildet ist.

5. Wundauflage nach einem oder mehreren der vorstehenden Ansprüche, **dadurch gekennzeichnet, dass** das Flächengewicht der Faservliesschicht (12) 15 - 100g/m², insbesondere 20 - 60 g/m², insbesondere 25 - 40 g/m² beträgt.

6. Wundauflage nach einem oder mehreren der vorstehenden Ansprüche, **dadurch gekennzeichnet, dass** die Dichte der Faservliesschicht (12) 10 - 1000 kg/m³, insbesondere 50 - 250 kg/m³, insbesondere 100 - 150 kg/m³ beträgt.

7. Wundauflage nach einem oder mehreren der vorstehenden Ansprüche, **dadurch gekennzeichnet, dass** die flüssigkeitsundurchlässige Kunststofffilmschicht (16) aus einem thermoplastischen Material, insbesondere aus Polyolefin, insbesondere aus Polypropylen, gebildet ist.

8. Wundauflage nach einem oder mehreren der vorstehenden Ansprüche, **dadurch gekennzeichnet, dass** die flüssigkeitsundurchlässige Kunststofffilmschicht (16) ein Flächengewicht von 5 - 100g/m², insbesondere von 8 - 50 g/m², insbesondere von 10 - 25 g/m², und eine Dicke von 5 - 100 µm, insbesondere von 8 - 50 µm, insbesondere von 10 - 25 µm aufweist.

9. Wundauflage nach einem oder mehreren der vorstehenden Ansprüche, **dadurch gekennzeichnet, dass** das Flächengewicht des Faseranteils des Saug/Spülkörpers (4) 20 - 500 g/m², insbesondere 30 - 300 g/m², insbesondere 30 - 200 g/m², insbesondere 30 - 150 g/m² beträgt.

10. Wundauflage nach einem oder mehreren der vorstehenden Ansprüche, **dadurch gekennzeichnet, dass** die Dichte des Faseranteils des Saug/Spülkörpers (4) 20 - 500 kg/m³, insbesondere 30 - 300 kg/m³, insbesondere 50 - 200 kg/m³ beträgt.

11. Wundauflage nach einem oder mehreren der vorstehenden Ansprüche, **dadurch gekennzeichnet, dass** der Bedeckungsgrad der partiell und strukturiert aufgebrachten atraumatisch wirkenden Beschichtung (22) 20 - 70 %, insbesondere 25 - 50 %, insbesondere 30 - 40 % beträgt.

12. Wundauflage nach einem oder mehreren der vorstehenden Ansprüche, **dadurch gekennzeichnet, dass** die partiell und strukturiert aufgebrachte atraumatisch wirkende Beschichtung (22) streifenförmig ausgebildet ist.

13. Wundauflage nach Anspruch 12, **dadurch gekennzeichnet, dass** die Breite der Streifen (24) 1 - 3 mm beträgt und/oder dass der Abstand der Streifen (24) voneinander 4 - 8 mm, insbesondere 4 - 6 mm beträgt.

14. Wundauflage nach einem oder mehreren der vorstehenden Ansprüche, **dadurch gekennzeichnet, dass** das Flächengewicht des Faseranteils des faservliesbasierten Saug-/Spülkörpers (4) 30 - 100 g/m² beträgt und dass das Flächengewicht des superabsorbierenden Materials innerhalb des faservliesbasierten Saug-/Spülkörpers (4) 50 - 100 g/m² beträgt und dass die Dicke der Wundauflage im mit salzhaltiger wässriger Lösung beaufschlagten Zustand 3 - 7 mm, insbesondere 4 - 6 mm beträgt.

15. Verfahren zum Herstellen von Wundauflagen (2) nach einem oder mehreren der vorstehenden Ansprüche, **dadurch gekennzeichnet, dass** die Faservliesschicht (12), die flüssigkeitsundurchlässige Kunststofffilmschicht (16), eine den faservliesbasierten Saug-/Spülkörper (4) bildende Schicht und die Schicht (9) aus textilem Flächenmaterial als jeweils endlose Flachmaterialien zugeführt und in der genannten Schichtenfolge übereinander angeordnet werden und dass die Schichten nur entlang ihres Umfangsrands (18) miteinander, insbesondere durch Ultraschall- oder Laserschweißen, verbunden werden und dabei die Wundauflagen (2) aus den Flachmaterialien vereinzelt werden.

## Claims

1. A wound dressing (2) for wound treatment in a moist or moist/wet environment, with a fibre nonwoven-based suction/rinsing body (4) in which superabsorbent material is incorporated in a distributed manner, a saline aqueous solution, especially Ringer's solution, which preferably contains a substance with antimicrobial action, having been applied to the suction/rinsing body (4) by the manufacturer, and with a cover (6) forming the outer visual sides of the wound dressing, the cover (6) comprising on the wound-facing side of the wound dressing a layer (9) composed of a textile planar material, especially composed of a weft-knitted fabric, warp-knitted fabric or woven fabric, the layer (9) composed of a textile planar material having on the wound-facing side an atraumatically acting coating (22), preferably composed of silicone, which has been applied on the outer side in a partial and structured manner, having a degree of coverage of at most 70%, **characterized in that** the cover (6) comprises a fibre nonwoven layer (12) on the side of the wound dressing that faces away from the wound, which fibre nonwoven layer (12) forms the outer visual side of the wound dressing that faces away from the wound, and **in that** a fluid-impermeable plastics film layer (16) is arranged on the wound-facing side of said fibre nowoven layer (12), and **in that** the fibre nonwoven layer (12), the plastics film layer (16), the suction/rinsing body (4) and the layer (9) composed of textile planar material are not joined to another in a planar manner, but are in contact with one another via their planar sides in a loose and slidable manner, the fibre nowoven layer (12), the plastics film layer (16), the suction/rinsing body (4) and the layer (9) composed of textile planar material being joined to one another only along their peripheral edge (18) by means of a joint.

2. The wound dressing as claimed in claim 1, **characterized in that** the joint is an ultrasonic welded joint.

3. The wound dressing as claimed in claim 2, **characterized in that** the ultrasonic welded joint comprises a succession of discrete ultrasonic welded points.

4. The wound dressing as claimed in one or more of the preceding claims, **characterized in that** the fibre nonwoven layer (12) is formed from thermoplastic fiber material, especially from polyolefin, especially from polypropylene.

5. The wound dressing as claimed in one or more of the preceding claims, **characterized in that** the surface weight of the fibre nonwoven layer (12) is 15-100 g/m², especially 20-60 g/m², especially 25-40 g/m².

6. The wound dressing as claimed in one or more of the preceding claims, **characterized in that** the density of the fibre nonwoven layer (12) is 10-1000 kg/m³, especially 50-250 kg/m³, especially 100-150 kg/m³.

7. The wound dressing as claimed in one or more of the preceding claims, **characterized in that** the fluid-impermeable plastics film layer (16) is formed from a thermoplastic material, especially from polyolefin, especially from polypropylene.

8. The wound dressing as claimed in one or more of the preceding claims, **characterized in that** the fluid-impermeable plastics film layer (16) has a surface weight of 5-100 g/m², especially of 8-50 g/m², especially of 10-25 g/m², and a thickness of 5-100 µm, especially of 8-50 µm, especially of 10-25 µm.

9. The wound dressing as claimed in one or more of the preceding claims, **characterized in that** the surface weight of the fiber fraction of the suction/rinsing body (4) is 20-500 g/m², especially 30-300 g/m², especially 30-200 g/m², especially 30-150 g/m².

10. The wound dressing as claimed in one or more of the preceding claims, **characterized in that** the density of the fiber fraction of the suction/rinsing body (4) is 20-500 kg/m³, especially 30-300 kg/m³, especially 50-200 kg/m³.

11. The wound dressing as claimed in one or more of the preceding claims, **characterized in that** the degree of coverage of the coating (22) which has been applied in a partial and structured manner and which acts atraumatically is 20-70%, especially 25-50%, especially 30-40%.

12. The wound dressing as claimed in one or more of the preceding claims, **characterized in that** the coating (22) which has been applied in a partial and structured manner and which acts atraumatically is in the form of strips.

13. The wound dressing as claimed in claim 12, **characterized in that** the width of the strips (24) is 1-3 mm and/or **in that** the distance of the strips (24) from one another is 4-8 mm, especially 4-6 mm.

14. The wound dressing as claimed in one or more of the preceding claims, **characterized in that** the surface weight of the fiber fraction of the fibre nonwoven-based suction/rinsing body (4) is 30-100 g/m² and **in that** the surface weight of the superabsorbent material within the the fibre nonwoven-based suction/rinsing body (4) is 50-100 g/m² and **in that** the thickness of the wound dressing in the saline aqueous solution-applied state is 3-7 mm, especially 4-6 mm.

15. A method for producing wound dressings (2) as claimed in one or more of the preceding claims, **characterized in that** the fibre nonwoven layer (12), the fluid-impermeable plastics film layer (16), a layer forming the fibre nonwoven-based suction/rinsing body (4), and the layer (9) composed of textile planar material are fed as respectively continuous planar materials and arranged on top of one another in the stated sequence of layers and **in that** the layers are joined to one another only along their peripheral edge (18), especially by ultrasonic or laser welding, and, at the same time, the wound dressings (2) are singularized from the planar materials.

## Revendications

1. Pansement pour plaie (2) destiné au traitement des plaies en milieu humide ou humide-mouillé, comprenant un corps absorbant/de rinçage à base de non-tissé en fibres (4) dans lequel un matériau superabsorbant est reçu de manière distribuée, ledit corps absorbant/de rinçage (4) étant pourvu par le fabricant d'une solution aqueuse saline, en particulier la solution de Ringer, qui contient de préférence une substance ayant un effet antimicrobien, ainsi qu'une enveloppe (6) formant les faces visibles extérieures du pansement pour plaie, dans lequel ladite enveloppe (6) comprend, sur la face du pansement pour plaie laquelle montre vers la plaie, une couche (9) en un matériau textile en nappe, en particulier en un tricot, tissu à mailles ou tissu, dans lequel ladite couche (9) en un matériau textile en nappe sur la face montrant vers la plaie présente un revêtement (22) à effet atraumatique, de préférence en silicone, avec un degré de couverture de 70 % tout au plus, qui est appliqué partiellement et de manière structurée sur la face extérieure, **caractérisé par le fait que**, sur la face du pansement pour plaie laquelle montre dans la direction opposée à la plaie, ladite enveloppe (6) présente une couche de non-tissé en fibres (12) qui forme la face visible extérieure du pansement pour plaie laquelle montre dans la direction opposée à la plaie, et qu'une couche de film plastique (16) imperméable aux liquides est disposée sur la face de cette couche de non-tissé en fibres (12) laquelle montre vers la plaie, et que la couche de non-tissé en fibres (12), la couche de film plastique (16), le corps absorbant/de rinçage (4) et la couche (9) en matériau textile en nappe ne sont pas reliés entre eux en nappe, mais reposent de manière lâche et déplaçable l'un contre l'autre par leurs côtés plats, la couche de non-tissé en fibres (12), la couche de film plastique (16), le corps absorbant/de rinçage (4) et la couche (9) en matériau textile en nappe ne sont reliés entre eux que le long de leur bord périphérique (18) par une liaison par assemblage.

2. Pansement pour plaie selon la revendication 1, **caractérisé par le fait que** la liaison par assemblage est une liaison de soudage par ultrasons.

3. Pansement pour plaie selon la revendication 2, **caractérisé par le fait que** la liaison de soudage par ultrasons comprend une succession de points discrets de soudage par ultrasons.

4. Pansement pour plaie selon une ou plusieurs des revendications précédentes, **caractérisé par le fait que** la couche de non-tissé en fibres (12) est réalisée à partir de matériau fibreux thermoplastique, en particulier de polyoléfine, en particulier de polypropylène.

5. Pansement pour plaie selon une ou plusieurs des revendications précédentes, **caractérisé par le fait que** le grammage de la couche de non-tissé en fibres (12) est compris entre 15 et 100 g/m², en particulier entre 20 et 60 g/m², en particulier entre 25 et 40 g/m².

6. Pansement pour plaie selon une ou plusieurs des revendications précédentes, **caractérisé par le fait que** la densité de la couche de non-tissé en fibres (12) est comprise entre 10 et 1000 kg/m³, en particulier entre 50 et 250 kg/m³, en particulier entre 100 et 150 kg/m³.

7. Pansement pour plaie selon une ou plusieurs des revendications précédentes, **caractérisé par le fait que** la couche de film plastique (16) imperméable aux liquides est réalisée à partir d'un matériau thermoplastique, en particulier de polyoléfine, en particulier de polypropylène.

8. Pansement pour plaie selon une ou plusieurs des revendications précédentes, **caractérisé par le fait que** la couche de film plastique (16) imperméable aux liquides présente un grammage compris entre 5 à 100 g/m², en particulier entre 8 et 50 g/m², en particulier entre 10 et 25 g/m², ainsi qu'une épaisseur comprise entre 5 et 100 µm, en particulier entre 8 et 50 µm, en particulier entre 10 et 25 µm.

9. Pansement pour plaie selon une ou plusieurs des revendications précédentes, **caractérisé par le fait que** le grammage de la partie fibreuse du corps absorbant/de rinçage (4) est compris entre 20 et 500 g/m², en particulier entre 30 et 300 g/m², en particulier entre 30 et 200 g/m², en particulier entre 30 à 150 g/m².

10. Pansement pour plaie selon une ou plusieurs des revendications précédentes, **caractérisé par le fait que** la densité de la partie fibreuse du corps absorbant/de rinçage (4) est comprise entre 20 et 500 kg/m³, en particulier entre 30 et 300 kg/m³, en particulier entre 50 et 200 kg/m³.

11. Pansement pour plaie selon une ou plusieurs des revendications précédentes, **caractérisé par le fait que** le degré de couverture du revêtement (22) à effet atraumatique appliqué partiellement et de manière structurée est compris entre 20 et 70 %, en particulier entre 25 et 50 %, en particulier entre 30 et 40 %.

12. Pansement pour plaie selon une ou plusieurs des revendications précédentes, **caractérisé par le fait que** le revêtement (22) à effet atraumatique appliqué partiellement et de manière structurée est réalisé en forme de bande.

13. Pansement pour plaie selon la revendication 12, **caractérisé par le fait que** la largeur des bandes (24) est comprise entre 1 et 3 mm et/ou que la distance séparant les bandes (24) les unes des autres est comprise entre 4 et 8 mm, en particulier entre 4 et 6 mm.

14. Pansement pour plaie selon une ou plusieurs des revendications précédentes, **caractérisé par le fait que** le grammage de la partie fibreuse du corps absorbant/de rinçage (4) à base de non-tissé en fibres est compris entre 30 et 100 g/m² et que le grammage du matériau superabsorbant au sein du corps absorbant/de rinçage (4) à base de non-tissé en fibres est compris entre 50 et 100 g/m² et que l'épaisseur du pansement pour plaie, lorsqu'il est à l'état où il est exposé à une solution aqueuse saline, est comprise entre 3 et 7 mm, en particulier entre 4 et 6 mm.

15. Procédé de fabrication de pansements pour plaie (2) selon une ou plusieurs des revendications précédentes, **caractérisé par le fait que** la couche de non-tissé en fibres (12), la couche de film plastique (16) imperméable aux liquides, une couche formant le corps absorbant/de rinçage (4) à base de non-tissé en fibres et la couche (9) en matériau textile en nappe sont fournies chacune en tant que matériaux plats sans fin et sont disposées les unes au-dessus des autres dans la succession de couches susmentionnée et que les couches ne sont reliées entre elles que le long de leur bord périphérique (18), en particulier par soudage par ultrasons ou laser, et, ce faisant, les pansements pour plaie (2) composés des matériaux plats sont séparés.
